# EUROPEAN PATENT APPLICATION

(11) **EP 3 162 418 A1**
(43) Date of publication of application: **03.05.2017**
(21) Application number: 16196303.8
(22) Date of filing: 28.10.2016
(51) Int. Cl.: A63H 3/00, A63H 3/02, A61M 21/00, G04C 19/02, A63H 33/26, A63H 3/28, A63H 3/36

(54) **SOUND MODULE FOR A TOY**

(30) Priority: 29.10.2015 GB 201519146
(71) Applicant: Golden Bear Products Limited, Telford, Shropshire TF1 4EY (GB)
(72) Inventor: NICHOLLS, Christine Anne, Penkridge, Shropshire ST19 5QP (GB); MAPP, David John, Shewsbury, Shropshire SY3 0LW (GB); HUGHES, John Barry, Much Wenlock, Shropshire TF13 6JQ (GB)
(74) Representative: Gunning, Jack Peter

(57) **Abstract**

There is provided a sound module for a toy, the sound module including: a controller; a speaker operatively connected to the controller; and a light operatively connected to the controller. The sound module is operable in at least a first mode and a second mode. In the first mode the controller controls the speaker to provide entertaining noises. In the second mode the controller: controls the light to provide calming illumination and/or controls the speaker to provide calming noises for a first period; and then controls the light to provide invigorating illumination and/or controls the speaker to provide invigorating noises for a second period. There is also provided a toy including the sound module.

## Description

### Description of Invention

This invention relates to a sound module for a toy and a toy including the sound module. More particularly, this invention relates to a sound module having a first play mode and a second sleep aid mode.

The invention is defined by the claims.

There is provided a sound module for a toy, the sound module including:
a controller;
a speaker operatively connected to the controller;
a light operatively connected to the controller; and
a child operable button operatively connected to the controller
wherein the sound module is operable in at least a first mode and a second mode;
wherein in the first mode the controller controls the speaker to provide entertaining noises; and
wherein in the second mode the controller:
controls the light to provide calming illumination and/or controls the speaker to provide calming noises for a first period; and then
controls the light to provide invigorating illumination and/or controls the speaker to provide invigorating noises for a second period;
wherein in the second mode between the first period and the second period the controller:
   controls the light to provide no illumination and the speaker to provide no noise for a third period; and
   wherein in response to actuation of the button in the second mode the controller interrupts the third period and controls the light to provide calming illumination and/or controls the speaker to provide calming noises for an additional period.

Such a sound module can be advantageously used in a toy which is both a play thing and a sleep aid. In the first day mode the sound module functions as a toy and in the second sleep mode the sound module functions as a sleep aid. As a child may play with the toy in the first mode and become familiar with it, the toy including such a sound module is particularly effective as a sleep aid due to the comfort provided to a child by a familiar toy.

There is also provided a sound module for a toy, the sound module including: a controller;
a speaker operatively connected to the controller; and
a light operatively connected to the controller;
wherein the sound module is operable in at least a first mode and a second mode;
wherein in the first mode the controller controls the speaker to provide entertaining noises; and
wherein in the second mode the controller:
controls the light to provide calming illumination and/or controls the speaker to provide calming noises for a first period; and then
controls the light to provide invigorating illumination and/or controls the speaker to provide invigorating noises for a second period.

Such a sound module can also be advantageously used in a toy which is both a play thing and a sleep aid.

The second period may include the controller first controlling the light to provide invigorating illumination; and then, after an intervening period, the controller controls the speaker to provide invigorating noises.

In the second mode between the first period and the second period the controller may control the light to provide no illumination and the speaker to provide no noise for a third period.

The sound module may further include a child operable button operatively connected to the controller, wherein in response to actuation of the button in the second mode the controller interrupts the third period and controls the light to provide calming illumination and/or controls the speaker to provide calming noises for an additional period.

The button and the light may be integrally formed.

The sound module may further include a display operatively connected to the controller, wherein the controller controls the display to display the current time and/or the time the second period is due to commence and/or the time until the second period is due to commence.

The sound module may comprise a formation which enables it to stand upon a surface and the light projects towards a desired location, e.g. towards a child.

The entertaining noises may include catchphrases, songs, and/or educational information.

The calming noise may be selected from at least one of the group consisting of wave sounds, white noise, insect cricket sounds, water fall sounds, heartbeat sounds and rain sounds.

The sound module may further include the features of the dependent claims.

There is also provided a toy including the sound module.

The sound module may be within the toy. The toy may include a region for allowing sound from the speaker to escape from the toy and/or a region for allowing illumination from the light to escape from the toy.

The sound module may be removable from the toy.

The toy may be a plush toy.

The toy may also further include the features of the dependent claims.

Embodiments of the invention will now be described with reference to the accompanying drawings, of which:
FIGURE 1 is a front view of a sound module in accordance with an embodiment of the invention;
FIGURE 2 is a rear view of the sound module of figure 1;
FIGURE 3 is a front view of a toy including the sound module of figure 1; and
FIGURE 4 is a rear view of the toy of figure 3.

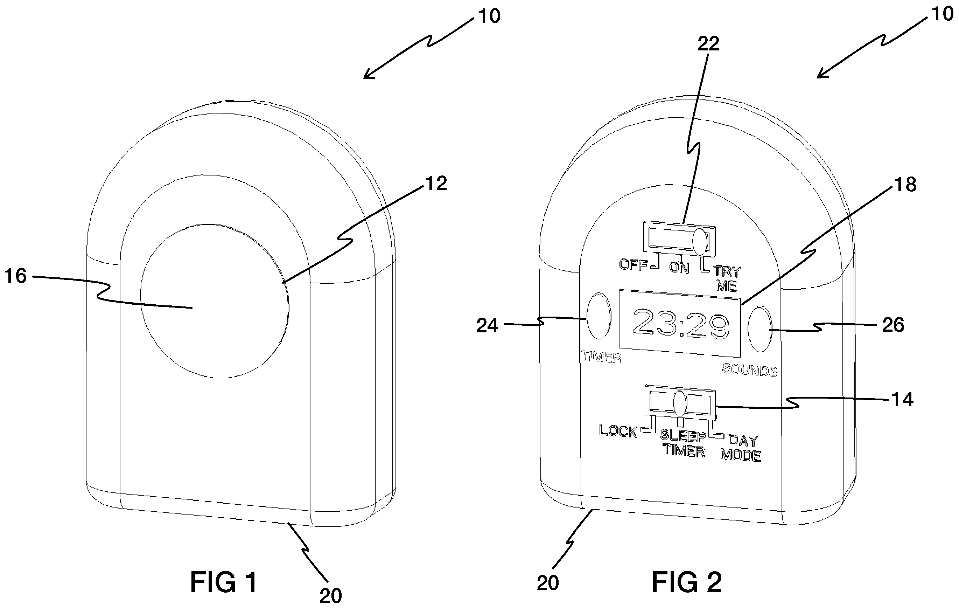

Referring firstly to figure 1, there is shown a sound module in accordance with the present invention, indicated generally at 10. The sound module 10 includes a controller (not shown), a speaker operatively connected to the controller (not shown), and a light 12 also operatively connected to the controller.

The sound module 10 is operable in at least a first mode and a second mode. The first mode is a day mode and the second mode is a sleep mode. The day and sleep modes are selected using a switch 14. In the first day mode the controller controls the speaker to provide entertaining noises. The entertaining noises can include catchphrases, songs and educational information, as is known in the art of toys.

In the second sleep mode the controller controls the light 12 to provide calming illumination and controls the speaker to provide calming noises for a first period. This illumination and noise can help a child to fall asleep. Children often have difficulty falling asleep without attention from their parents. The illumination and noises can therefore free a parent from having to assist their child in falling asleep. Red and yellow illumination has been found to be particularly effective in helping children to fall asleep. In particular, red illumination is believed to replicate the light of a womb to which children have at least a subconscious memory and it has therefore been found to be comforting to them. Noises which have been found to be particularly calming, and therefore sleep inducing to children, include wave sounds, white noise, insect cricket sounds, waterfall sounds, heartbeat sounds and rain sounds. In particular, heart beat sounds are again familiar to small children from their time *in utero.*

The controller subsequently (not necessarily immediately, as explained in more detail below) controls the light 12 to provide invigorating illumination and/or controls the speaker to provide invigorating noises for a second period. The invigorating illumination may be blue or green light. The invigorating noises may be a traditional alarm sound, for instance, an intermittent siren.

In particular, the controller may first control the light 12 to provide invigorating illumination and then after an intervening period, the controller controls the speaker to provide invigorating noises. In this way a child may be given a two stage signal. In the first stage the light 12 indicates that it is permissible for the child to wake up and in the second stage, after the intervening period, the alarm sounds and wakes up the child if he is still asleep. This arrangement may be particularly useful with children who are able to get out of bed. In particular, the child is made aware that it is permissible to get of bed when the invigorating illumination is provided but not necessarily awoken by the illumination. Therefore, the child can determine whether it is permissible for him to get out of bed or not by observing the light 12. This can be useful as children frequently do not understand what time to get out of bed, as they are unable to tell the time from a conventional clock. After the intervening period the controller provides a conventional alarm (by controlling the speaker to provide invigorating noises).

After the provision of calming illumination and/or noises for a first period the controller controls the light 12 to provide no illumination and the speaker to provide no noise for a third period, in this period the child is asleep. Accordingly, it is not necessary to provide calming illumination or calming noises.

The sound module 10 also includes a child operable button 16 operatively connected to the controller. In response to actuation of the button 16 in the second mode the controller interrupts the third period and controls the light 12 to provide calming illumination and controls the speaker to provide calming noises for an additional period. Such a feature is particularly advantageous when child awakens in the middle of the night and requires comforting in order to return to sleep. All a child needs do is operate the button 16 to receive calming and comforting illumination and noises and so return to sleep.

In response to actuation of the button 16 in the second mode the controller may control the light to provide invigorating illumination for the second period.

In this way the light is off until a child operates the button 16 and only provides invigorating illumination for the second period when the button is pressed. This can prevent the light from waking up a child if he is sleeping, which may be undesirable, e.g. where an adult responsible for the child desires the child to have as much sleep as possible.

The button 16 and the light 12 are integrally formed. Accordingly, pressing the button 16 causes the button 16 to illuminate, which can help associate operation of the button 16 with the calming effect in the mind of the child.

The sound module 10 also includes a display 18 operatively connected to the controller. The controller controls the display 18 to display the current time and/or the time the second period is due to commence and/or the time until the second period is due to commence. This enables an adult to interact with the sound module and control the various time periods, as will be described in more detail below.

The sound module 10 includes a formation, specifically a flat bottom surface 20, which enables the sound module 10 to stand upright on a surface (not shown). This enables the sound module 10 to be placed near a child such that the illumination from the light 12 is visible to the child user. Alternative arrangements may be used, for example, the sound module 10 may comprise discrete feet e.g. rubberised feet. The feet may be in a single plane. This enables the light to project towards a desired location, e.g. towards a child.

In use, the sound module 10 is operated as follows. First, the on/off switch 22 is moved to the on position.

The on/off switch 22 also includes a "try me" position which may be used to demonstrate the sound module, for example, in a toy shop. Such "try me" modes are particularly useful in the sale of toys and may be used, in particular, to attract the attention of would be purchasers or to encourage a purchase by an interested customer. Such "try me" modes and sales techniques are well known in the art and will not be described further.

The switch 14 is then used to select either the first day mode or the second sleep mode. In the first day mode the sound module functions as a sound module for a conventional toy, e.g. a conventional plush toy, providing entertaining noises including catchphrases, songs and/or educational information, as is well known *per se.*

In the sleep mode a time can be set by pressing and holding the button 24 to increase the time between the present time and the time when the child is due to awaken, that is, the time at which the light 12 provides invigorating illumination and the speaker provides invigorating noises. Then, if desired, the adult operator may press the sounds button 26 to select the differing calming noises described above which are provided for the first period.

The display 18 first displays the current time and then displays either the time at which the second period is due to commence, e.g. 7 am, or the time until the second period is due to commence, e.g. 1 hour. The second alternative may be particularly advantageous for a child having a nap which may not always commence at the same time of day. For example, it may be desired to put a child down for a nap for, say, 1 hour at a number of times throughout the day. This second alternative is therefore easier to use because the adult using the device does not have to work out the current time plus the length of time of the desired nap.

The switch 14 is then moved to lock position, in this position the sound module 10 is still in the first sleep mode but the buttons 24, 26 do not function. This hinders the child from adjusting the settings which the adult has chosen.

The controller controls the light 12 to provide calming illumination and controls the speaker to provide calming noises for a first period, typically 15 minutes. The sound module 10 is then left adjacent the child that it is desired to go to sleep and the illumination and noise assist the child in falling asleep.

If the child wakes the child can press the button 16, which is operable even in the lock position, and he will receive the calming illumination and calming noises for an additional period, typically 5 minutes. This is often long enough for the child to return to sleep should he stir in the night. As the button 16 is relatively large the child is be able to operate the button 16 relatively easily, for example by hugging a toy 50 in which the sound module is included (as described in more detail below).

After the third period has elapsed and it is time for the child to awaken the light 12 provides invigorating illumination. During this period it is permitted for the child to get out of bed, if they are of that age, although the sound module 10 does not actively awaken the child. Then, after an intervening period, the controller controls the speaker to provide invigorating noises and awaken the child.

When the switch 14 is in the first day mode the controller controls the speaker to provide entertaining noises, as mentioned above. The entertaining noises may be provided in response to actuation of the button 16, alternatively the noises may be provided in response to some other actuation means, for example a movement sensor, a microphone, and other alternatives which will be apparent to the skilled person.

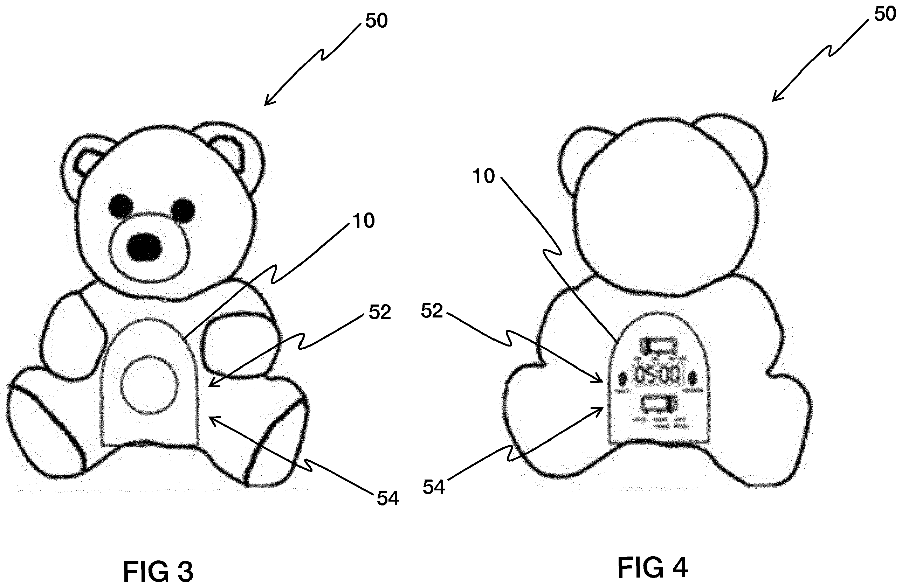

As shown in figures 3 and 4 the sound module 10 may be included in a toy 50. The toy 50 illustrated in figures 3 and 4 is a plush toy, specifically a teddy. The toy 50 includes a region 52 for allowing sound from the speaker to escape from the toy 50 and a region 54 for allowing illumination from the light 12 to escape from the toy 50. The toy may be another kind of plush toy, for example a sheep. Alternatively, the toy may be a non-plush toy, for example a fire engine or a train.

As will be apparent, if the sound module 10 is not within the toy but instead sits on an outer surface of the toy, it is not necessary to include the regions for allowing the sound and light 12 to escape from the toy.

The sound module 10 is removable from the toy 50. This means that the sound module 10 may be sold separately to the toy 50 and additionally that the toy 50 may be cleaned with the sound module 10 removed, for instance in a washing machine.

The invigorating noises may be noises related to the type or character of the toy 50 in which the sound module 10 is included. For example, the invigorating noises may be those noises typically associated with teddy bears, sheep or fire engines, as appropriate.

The toy may further include a mounting, e.g. a strap, for attaching the toy to, for example, the sides of a cot.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A sound module for a toy, the sound module including:
a controller;
a speaker operatively connected to the controller;
a light operatively connected to the controller; and
a child operable button operatively connected to the controller,
wherein the sound module is operable in at least a first mode and a second mode;
wherein in the first mode the controller controls the speaker to provide entertaining noises; and
wherein in the second mode the controller:
controls the light to provide calming illumination and/or controls the speaker to provide calming noises for a first period; and then
controls the light to provide invigorating illumination and/or controls the speaker to provide invigorating noises for a second period;wherein in the second mode between the first period and the second period the controller:
controls the light to provide no illumination and the speaker to provide no noise for a third period; and
wherein in response to actuation of the button in the second mode the controller interrupts the third period and controls the light to provide calming illumination and/or controls the speaker to provide calming noises for an additional period.

2. The sound module according to claim 1, wherein the second period includes:
the controller first controlling the light to provide invigorating illumination;
and then, after an intervening period, the controller controls the speaker to provide invigorating noises.

3. The sound module according to claims 1 or 2, wherein the button and the light are integrally formed.

4. The sound module according to any preceding claim, further including a display operatively connected to the controller, wherein the controller controls the display to display the current time and/or the time the second period is due to commence and/or the time until the second period is due to commence.

5. A sound module according to any preceding claim, wherein the sound module comprises a formation which enables it to stand upon a surface and the light projects towards a desired location, e.g. towards a child.

6. The sound module according to any preceding claim, wherein the entertaining noises include catchphrases, songs, and/or educational information.

7. The sound module according to any preceding claim, wherein the calming noise is selected from at least one of the group consisting of wave sounds, white noise, insect cricket sounds, water fall sounds, heartbeat sounds and rain sounds.

8. A toy including the sound module according to any preceding claim.

9. A toy according to claim 8, wherein the sound module is within the toy and the toy includes a region for allowing sound from the speaker to escape from the toy and a region for allowing illumination from the light to escape from the toy.

10. The toy according to claim 8 or claim 9, wherein the sound module is removable from the toy.

11. The toy according to any of claims 8 to 10, wherein the toy is a plush toy.
